# EUROPEAN PATENT SPECIFICATION

(11) **EP 0 470 007 B1**
(45) Date of publication and mention of the grant of the patent: **15.03.1995**
(21) Application number: 91402183.7
(22) Date of filing: 02.08.1991
(51) Int. Cl.: A61L 15/48, A61L 15/24

(54) **Wound-covering materials**
Wundabdeckmaterial
Matériau pour le recouvrement des plaies

(30) Priority: 03.08.1990 JP 204996/90; 27.09.1990 JP 255272/90
(43) Date of publication of application: 05.02.1992
(73) Proprietor: TERUMO KABUSHIKI KAISHA, Tokyo 151 (JP)
(72) Inventor: Koide, Mikio, c/o Terumo Kabushiki Kaisha, Kanagawa-Ken (JP); Onishi, Makoto, c/o Terumo Kabushiki Kaisha, Kanagawa-Ken (JP); Seita, Yukio, c/o Terumo Kabushiki Kaisha, Kanagawa-Ken (JP); Katakura, Takeo, c/o Terumo Kabushiki Kaisha, Kanagawa-Ken (JP); Takahasi, Akira, c/o Terumo Kabushiki Kaisha, Kanagawa-Ken (JP)
(74) Representative: Gillard, Marie-Louise

(56) References cited:
- EP-A- 0 184 392
- EP-A- 0 336 543

## Description

### Background of the Invention

### 1. Field of the Invention

This invention relates to improved wound-covering materials. More particularly, the invention is concerned with wound-covering materials with a hydrophilic surface. The present wound-covering materials are applied to a site of skin defect when the skin is defectively injured by wounding, burn and the like.

### 2. Description of the Prior Art

There have been known so-called dry dressing and wet dressing as a method and material for treating wounds including traumatic dermal wound and wound caused by removal of skin for dermatoplasty as well as wound associated with disease. In the former, healing is achieved by maintaining the wounded site dry until a crust is formed. The latter is directed to creation of adequately wet circumstances to enable floating of epidermic cells. It is recognized in the latter method that more rapid healing of the wound is accomplished, less dry necrosis is formed and a protective effect on the wound surface is produced.

It is pointed out, however, as to the use of a surgical drape, one of the wet dressing techniques that there is retention of a large amount of exudate which is possibly reabsorbed through the wound surface, risk of infection is high and direct contact of the adhesive with the wound surface may be detrimental to healing of the wound. The drape is also apt to be separated from the wound surface. A drape having small projections attached to the surface to be in contact with wound surface is available, but oppositely, it is difficult to remove.

In order to overcome these problems there have recently been made available covering materials using a biopolymer such as collagen, chitin or fibrin on the area that can be in contact with the wounded site or those in which a moisture-retaining ingredient is dispersed in the rubber base material in order to achieve close contact, non-adhesion and maintenance of highly aqueous state. In addition, wound-covering materials previously proposed by us are available which comprise a biocompatible hydrogel-forming support layer (for example, of carboxymethylcellulose, arginate, hyaluronate or poly(meth)acrylate) in which at least a portion of the area to be in contact with the wounded site is coated with a water-repellent substance and a moisture permeation-adjusting layer which is formed on the side opposite to the area of said support layer to be in contact with the wounded site (Japanese Patent Application Laid-Open-to-Public No. 183760/1987). All of the above-cited materials have achieved their purpose to some extent.

On the other hand, porous macromolecular membranes are available as the dry dressing. Since such membranes would lead to fixation of the dressing on the wound surface, which will cause blooding when detached, dressings in which a non-adhesive porous film (Melolin®, Smith & Nephew Limited) is used on the area to be in contact with the wound surface have recently become commercially available.

Although the prior art covering materials as mentioned above are effective to some extent, there is a problem of physical property in use as a wound-covering material in that the material used in forming a biocompatible base layer in the area to be in contact with the wounded site is apt to undergo degradation or detachment. Furthermore, the matters from the degradation or the detachment are possibly be recognized as a foreign matter with the risk of delaying healing of the wound. Further, although the moisture permeation-adjusting layer formed on the opposite side of the area of the support layer to be in contact with wound is required to possess an adequate moisture-permeating capacity and a capacity of inhibiting microbial attack, a considerably limited scope of materials meet both of the requirements.

It is therefore an object of the invention to provide wound-covering materials which, by coating with a nonionic surface-active agent or an amino-acid surface-active agent the surface and the inner surfaces of the pores of a hydrophobic porous membrane to make them hydrophilic, are capable of maintaining good contact with the wound surface, are not easily be degraded or detached when contacted with wound, and are capable of facilitating healing of wound, especially regeneration of the epidermis to enable rapid therapy of the wound and are simultaneously provided with moisture permeability by the hydrophilic porous membrane.

Heretofore, porous membranes having superior membrane strength and separation capacity have been prepared by the treatment that makes a hydrophobic membrane hydrophilic. As the treatment for that purpose are known the technique as disclosed in Japanese Patent Application Laid-Open-to-Public No. 93734/1983 in which hydrophilic groups are provided on the surface of the membrane by a treatment with an aqueous solution of an alkali such as NaOH or KOH and the technique as disclosed in Japanese Patent Application Laid-Open-to-Public No. 17978/1979 in which a hydrophobic porous membrane is immersed in an alcohol and treated with an aqueous solution of a water-soluble polymer followed by drying, and the water-soluble polymer remaining on the surface is subjected to an insolubilization treatment such as heat irradiation.

However, the prior art treatments of hydrophobic porous membranes to make them hydrophilic were encountered with such problems as described below. In the former method in which hydrophilic groups are provided on the surface by treatment with an aqueous alkali solution, there is a possibility for the membrane strength to be reduced by the treatment. The method which comprises treatment with an aqueous solution of a water-soluble polymer followed by drying and insolubilization treatment by radiation takes a long period of time from the alcohol immersion through the substitution with an aqueous polymer solution and is possibly associated with reduction of the membrane strength on insolubilization treatment as well as decrease in separation capacity by the change of the diameter of the pores of the membrane.

### Summary of the Invention

The above-mentioned object is achieved by constituting a wound-covering material comprising a wound-covering material comprising a hydrophobic porous membrane, a surface and inner surfaces of the pores of the hydrophobic membrane being coated with a nonionic surface active agent or an amino-acid surface-active agent, the hydrophobic membrane being composed of a polyolefin or a halogenated polyolefin, the nonionic surface active agent being a propylene oxid/ethylene oxide block copolymer in which 60-90% by weight of the whole molecule is suitably occupied by an ethylene oxide chain, the amino-acid surface active agent being anionic and containing as a hydrophilic group an amino acid and as a hydrophobic group a fatty acid.

The hydrophobic porous membrane of the invention as mentioned above is prepared using as the raw material a polyolefin such as polypropylene, polyethylene or a copolymer of them, or a halogenated polyolefin such as polyvinylidene fluoride, a copolymer of vinylidene fluoride with propylene hexafluoride or a mixture of them. Pore diameter of the hydrophobic porous membrane is 0.01 - 1.0 »m on average.

The amino-acid surface-active agent contains as the hydrophilic group particularly L-glutamic acid and as the hydrophobic group a fatty acid.

The wound-covering materials of the invention are prepared, for example, by the procedures described below. The procedures comprise only immersing a base material for the hydrophobic porous membrane in a solution of a nonionic surface-active agent and drying it, which are very simple and convenient. The solvent may be any one that is capable of dissolving the nonionic surface-active agent and has affinity to the base material for the hydrophobic porous membrane but does not dissolve it. Although water may also be employed as the solvent, it is necessary in this case to immerse the hydrophobic porous membrane in an alcohol to effect water substitution for making it hydrophilic.

As the nonionic surface-active agent is preferably a propylene oxide/ethylene oxide block copolymer. The ethylene oxide chain of the copolymer preferably occupies 60-90% by weight of the whole molecule particularly in cases where said porous membrane is used for medical purposes for which safety is highly required. The solvent is preferably a lower alcohol such as methanol, ethanol or propanol. The product may be toxic at a level of the ethylene oxide below 60% by weight of the whole molecule and unsuitable for medical use. At a level over 90% by weight, affinity to hydrophobic substances will be poor due to a low proportion of the hydrophobic propylene oxide chain, which will make it difficult to uniformly fix the agent on the hydrophobic porous membrane and will result in producing a non-uniform membrane.

There is no limitation to the nature of the raw material for the base material of hydrophobic porous membranes. For example, fluorine polymers including polytetrafluoroethylene, polyvinylidene fluoride and copolymers or mixtures with other polymers are used. More particularly, polyolefin polymers such as polyethylene and polypropylene are employed.

The nonionic surface-active agent as mentioned above containing the ethylene oxide chain at a level of 60-90% by weight is of so high biological safety that dissolution of the entire amount used in such treatment would entirely be non-toxic. Accordingly, there is no need for the insolubilization treatment. When an amino-acid surface-active agent is used as the surface-active agent, the solvent is preferably a lower alcohol, particularly methanol. Use of other surface-active agents or other water-soluble hydrophilic nature-forming agents is much limited due to their toxicity. The scope of the hydrophobic porous membranes that can be used is limited because not only processing steps are very complicated but also reduction of membrane strength and other performances is possible even if the insolubilization treatment is feasible.

The wound-covering materials of a hydrophilic porous membrane treated with a nonionic surface-active agent or an amino-acid surface-active agent is prepared merely by a simple treatment of a hydrophobic porous membrane which needs none of the particular treatments with heat, radiation, an acid, an alkali and others as in the prior art methods. Accordingly, no reduction is induced of the membrane strength and other performances intrinsic of the hydrophobic porous base material and a variety of base materials may be used.

Furthermore, it is another object of the invention to provide wound-covering materials which are capable of maintaining good contact with the wound surface, not easily degraded or detached when contacted with wound, and capable of facilitating healing of wound, especially regeneration of the epidermis to enable rapid therapy of the wound and in which a porous polyolefin membrane is simultaneously provided with moisture permeability.

It is further object of the invention to provide wound-covering materials which are convenient for preventing external infection.

The above objects can be achieved by a wound-covering material described below.
(1) A wound-covering material comprising a hydrophobic membrane a surface of which is made hydrophilic
(2) The wound-covering material according to item 1 wherein the hydrophobic membrane is composed of a porous polyolefin or halogenated polyolefin and a surface of the membrane is chemically bound with a hydrophilic polymer
(3) The wound-covering material according to item 2 wherein pore diameter of the membrane is 0.01-1.0 »m on average
(4) The wound-covering material according to item 2 wherein steam permeability coefficient of the membrane is 200-5000 mg/m²/24 hrs.
(5) The wound-covering material according to any one of items 2-4 additionally comprising an antimicrobially active metal vapor-deposited on the surface of the membrane.

As described above, the wound-covering materials of the invention comprise a porous polyolefin membrane or a porous halogenated polyolefin membrane with a hydrophilic polymer chemically bound, which can facilitate regeneration of the epithelium due to hydration or wetting of the surface that is in contact with the adermic site.

The membranes used in the invention is a porous polyolefin membrane or a porous halogenated polyolefin membrane, which is preferably of polyethylene, polypropylene, polyvinylidene fluoride, polyvinylidene chloride, chlorinated polyethylene or the like in view of the common availability and low price. As the hydrophilic polymer to be bound to the porous polyolefin membrane or the porous halogenated polyolefin membrane is mentioned methoxyethyl acrylate or N-dimethylacrylamide. Polymethoxyethyl acrylate which is chemically bound to the above-mentioned polyolefin membrane by graft polymerization is advantageous due to its superior biocompatibility causing less foreign-matter reactions when contacted with biotissues. In this case, preferably, the membrane is subjected to a plasma-initiated surface graft polymerization with ethylacrylate to be elastic, before being subjected to a plasma-initiated surface graft polymerization with methoxyacrylate. The resulting covering materials suitably have a pore diameter in the range of 0.01-1.0 »m and a steam permeability coefficient in the range of 200-5000 mg/m²/24 hrs.

The metal used in the invention as the antimicrobial agent includes silver, copper, zinc or the like. Silver is preferred from an antimicrobial point of view. Two or more metals may be present on the membrane surface, which may be in element, oxide or salt. There is no particular limit to the amount of the metal contained in the porous membrane. It is preferable, however, in view of production cost and secondary pollution by the effluent metal, if any, to have the metal present on the membrane surface at a metal/carbon atomic ratio in the range of 0.02-5.0 as determined by X-ray photoelectronic spectrum. At a metal/carbon atomic ratio of over 5.0 the metal present on the membrane surface will be excessive so that the pore diameter will be reduced due to attached metal. This will result in loss of the steam permeability intrinsic of the membrane though being antimicrobially active. Excessive attachment of the metal could lead to formation of a metal film. This would not only result in blockade of the pores or peeling off of the metal layer on impact but also would economically be disadvantageous. At a metal/carbon atomic ratio of below 0.02, on the other hand, consistent antimicrobial activity could be lost.

The wound-covering materials of the invention are prepared, for example, as described below. First, predetermined amounts of liquid paraffin and a crystalline nucleus-forming agent are added to powdery polypropylene, and the mixture is melt kneaded to give pellets. The pellets are molten at 150-200°C and extruded using an extruder equipped with a T die. The extrudate is introduced into a cooling and fixing solution for cooling and fixing to a film. The liquid paraffin in the film is removed by extraction, and the resulting film is heat treated in air at around 135°C for approximately 2 min. to produce a porous polypropylene membrane. Said membrane is subjected to a plasma-initiated surface graft polymerization with methoxyethyl acrylate to give a porous polypropylene membrane that has been made hydrophilic. Surface lubricant properties of the membrane in wet state are shown in Table 1.

**Table 1**

| Surface lubricant properties of wound-covering materials | | | |
|---|---|---|---|
| Sample | Percent grafted (%) | Mean coefficient of friction (MIU) | Variation in coefficient of friction (MMD) |
| PP membrane | - | 0.315 | 0.0113 |
| PP-g-PMEA | 10 | 0.125 | 0.0040 |
| PP-g-PMEA | 25 | 0.120 | 0.0038 |
| PP-g-PMEA | 50 | 0.120 | 0.0037 |

Degree of surface lubrication of the surface of a material in wet state can be evaluated on the basis of mean coefficient of friction (frictional resistance) and variation in coefficient of friction (so-called rough feeling).

The mean coefficient of friction and the variation in coefficient of friction is respectively at a high level for the untreated polypropylene membrane. They are at a low level, respectively, on the contrary, when the membrane is treated with methoxyethyl acrylate to make it hydrophilic. The resulting membrane exhibits very low feeling of roughness in wet state, that is, it is slimy due to hydration.

Next, the porous membrane is placed in a bell jar for vacuum vapor deposition. Pressure in the bell jar is then reduced for a predetermined period of time to effect vacuum vapor deposition of silver. It is noted that metal may be contained on the membrane surface by any suitable technique. In addition to the above, sputtering, ion beam and other techniques may be employed.

On the porous membrane with a metal vapor deposited thereon as prepared above is spread an acrylic adhesive by means of a precisely coating device (applicator) to produce the wound-covering material according to the invention.

With the wound-covering material of the invention on the membrane surface of which a metal is present, growth of microorganisms is inhibited by antimicrobial activities of the metal.

### Description of the Preferred Embodiments

The invention will be described in more detail with reference to examples.

### Example 1

A porous polypropylene membrane base material 0.45 »m in average pore diameter and 90% in percent pore void was prepared and immersed in a 5% by weight methanol solution of a propylene oxide/ethylene oxide block copolymer surface-active agent containing 80% by weight of the ethylene oxide chain (For example, Pluronick F-68 (trade name), Asahi Denka Kogyo K.K.) for 30 sec. and dried in an oven at 60°C to prepare a hydrophilic porous membrane. The membrane uniformly absorbed water immediately when immersed in distilled water to indicate that it was highly hydrophilic.

### Example 2

The same porous membrane base material as in Example 1 was prepared, with which a propylene oxide/ethylene oxide block copolymer surface-active agent containing 10% by weight of the ethylene oxide chain (Pluronick L-101 (trade name), Asahi Denka Kogyo K.K.) was used under the same conditions as in Example 1 to give a hydrophilic porous membrane. The membrane instantly and uniformly absorbed water when immersed in distilled water to indicate that it was highly hydrophilic.

### Example 3

A porous polypropylene membrane base material 0.45 »m in average pore diameter and 90% in percent pore void was prepared and immersed in a 5% by weight methanol solution of a surface-active agent composed of monosodium N-lauryl-L-glutamate (LS-11, Ajinomoto Co., Inc.) and dried to give a hydrophilic porous membrane. The membrane exhibited good hydrophilic nature when immersed in distilled water.

### Example 4

A porous polyvinylidene fluoride membrane base material 0.45 »m in average pore diameter and 78% in percent pore void was prepared and immersed in a 5% by weight methanol solution of a propylene oxide/ethylene oxide block copolymer surface-active agent containing 80% by weight of the ethylene oxide chain (Pluronick F-68) for 30 sec. and dried in an oven at 60°C to prepare a hydrophilic porous membrane. The membrane uniformly absorbed water immediately when immersed in distilled water to indicate that it was highly hydrophilic.

### Example 5

A dermatoplastic test was carried out in rats on dermal defective wound using the hydrophilic porous membrane prepared according to Example 1.

A wound in 10/1000 inch in depth and ca. 2 x 2 cm in size was formed in the rat on the back skin using a dermatome (surgical device for removal of the skin). The surface was patched with a test specimen, and an untreated membrane was used as a control. The circumferential area was then pressure fixed. The animals were sacrificed at predetermined time intervals and autopsied.

Percent cuticularization up to day 3 was 89% with the hydrophilic porous membrane (n=6) and as low as 77% with the untreated porous membrane (n=6). Histologically, almost no inflammatory reaction was observed with the hydrophilic porous membrane. No epidermic hypertrophy was also observed.

### Example 6

A porous polypropylene membrane base material 0.45 »m in average pore diameter and 40% in percent pore void was prepared and immersed in a 5% by weight methanol solution of a propylene oxide/ethylene oxide block copolymer surface-active agent containing 80% by weight of the ethylene oxide chain (For example, Pluronick F-68 (trade name), Asahi Denka Kogyo K.K.) for 30 sec. and dried in an oven at 60°C to prepare a hydrophilic porous membrane. The membrane uniformly absorbed water immediately when immersed in distilled water to indicate that it was highly hydrophilic.

### Example 7

The same porous membrane base material as in Example 6 was prepared, with which a propylene oxide/ethylene oxide block copolymer surface-active agent containing 10% by weight of the ethylene oxide chain (Pluronick L-101 (trade name), Asahi Denka Kogyo K.K.) was used under the same conditions as in Example 1 to give a hydrophilic porous membrane. The membrane instantly and uniformly absorbed water when immersed in distilled water to indicate that it was highly hydrophilic.

### Example 8

A porous polypropylene membrane base material 0.45 »m in average pore diameter and 40% in percent pore void was prepared and immersed in a 5% by weight methanol solution of a surface-active agent composed of monosodium N-lauryl-L-glutamate (LS-11, Ajinomoto Co., Inc.) and dried to give a hydrophilic porous membrane. The membrane exhibited good hydrophilic nature when immersed in distilled water.

### Example 9

A porous polyvinylidene fluoride membrane base material 0.45 »m in average pore diameter and 40% in percent pore void was prepared and immersed in a 5% by weight methanol solution of a propylene oxide/ethylene oxide block copolymer surface-active agent containing 80% by weight of the ethylene oxide chain (Pluronick F-68) for 30 sec. and dried in an oven at 60°C to prepare a hydrophilic porous membrane. The membrane uniformly absorbed water immediately when immersed in distilled water to indicate that it was highly hydrophilic.

### Example 10

A dermatoplastic test was carried out in rats on dermal defective wound using the hydrophilic porous membrane prepared according to Example 6.

A wound in 10/1000 inch in depth and ca. 2 x 2 cm in size was formed in the rat on the back skin using a dermatome (surgical device for removal of the skin). The surface was patched with a test specimen, and an untreated membrane was used as a control. The circumferential area was then pressure fixed. The animals were sacrificed at predetermined time intervals and autopsied.

Percent cuticularization up to day 3 was 89% with the hydrophilic porous membrane (n=6) and as low as 77% with the untreated porous membrane (n=6). Histologically, almost no inflammatory reaction was observed with the hydrophilic porous membrane. No epidermic hypertrophy was also observed.

### Example 11

With 100 parts by weight of a mixture of polypropylenes (mixing ratio of 100:40), respectively having a melt flow index of 30 and 0.3 were melt kneaded 400 parts by weight of liquid paraffin (average molecular weight of 324) and 0.3 part by weight of 1,3,2,4-bis(p-ethylbenzylidene)-sorbitol as a crystalline nucleus-forming agent by means of a twin screw extruder to form pellets. The pellets were molten in said twin screw extruder at 150-200°C and extruded through a T die with a 0.6 mm slit into air to form filming. The filming was then introduced by means of a guide roller positioned right under the T die into a cooling and fixing solution from which the cooled and fixed filming was wound up. The wound filming was cut to a predetermined size, and the cut filming was fixed in both longitudinal and lateral directions and immersed in 1,1,2-trichloro-1,2,2-trifluoroethane four times for 10 min. each to extract the liquid paraffin in the filming. Then, the filming was heat treated in air at 135°C for 2 min. to produce a porous polypropylene membrane 0.6 »m in pore diameter and 140 »m in thickness.

The membrane was subjected to a plasma-initiated surface graft polymerization with methoxyethylacrylate to give a polypropylene membrane made hydrophilic.

### Example 12

With 100 parts by weight of a mixture of polypropylenes (mixing ratio of 100:40), respectively having a melt flow index of 30 and 0.3 were melt kneaded 400 parts by weight of liquid paraffin (average molecular weight of 324) and 0.3 part by weight of 1,3,2,4-bis(p-ethylbenzylidene)-sorbitol as a crystalline nucleus-forming agent by means of a twin screw extruder to form pellets. The pellets were molten in said twin screw extruder at 150-200°C and extruded through a T die with a 0.6 mm slit into air to form filming. The filming was then introduced by means of a guide roller positioned right under the T die into a cooling and fixing solution from which the cooled and fixed filming was wound up. The wound filming was cut to a predetermined size, and the cut filming was fixed in both longitudinal and lateral directions and immersed in 1,1,2-trichloro-1,2,2-trifluoroethane four times for 10 min. each to extract the liquid paraffin in the filming. Then, the filming was heat treated in air at 135°C for 2 min. to produce a porous polypropylene membrane 0.6 »m in pore diameter and 140 »m in thickness. The membrane was subjected to a plasma-initiated surface graft polymerization with ethylacrylate to be elastic.

Then the membrane was subjected to a plasma-initiated surface graft polymerization with methoxyethylacrylate to give the membrane made hydrophilic.

### Example 13

A solution comprising 18 parts by weight of powdery polyvinylidene fluoride (Kynar K301 manufactured by Mitsubishi Petrochemical Co., Ltd.) dissolved in 73.8 parts by weight of acetone and 8.2 parts by weight of dimethylformamide was cast on a polyethylene terephthalate film followed by immersion in a 1,1,2-trifluoroethane bath for 5 min. and drying. There was produced a porous polyvinylidene fluoride membrane 0.45 »m in average pore diameter and 135 »m in membrane thickness. The membrane was subjected to a plasma-initiated surface graft polymerization with methoxyethylacrylate to give a polyvinylidene fluoride membrane made hydrophilic.

### Example 14

The porous membrane obtained in Example 11 was vapor deposited by sputtering with silver. The vapor deposition by sputtering was performed first by placing the porous membrane in a bell jar for vapor deposition, reducing the pressure to 10⁻⁵ Torr, opening the shutter and sputtering silver on the porous membrane for a predetermined period of time.

For Examples 1-14, the membranes were substantially formed by one-layer porous membrane by observation of electromicroscope.

### Test Example 1

### (Assessment of antimicrobial activity)

Muller-Hilton agar (manufactured by Difco) was autoclaved, and the agar maintained at 50°C was divided in 20ml portions into dishes followed by allowing to stand at room temperature for one hour to solidify the agar. The microorganism was plate-cultured and suspended in a Tris buffer. The microbial suspension was applied by a swab three times throughout the medium. The test sample prepared by the procedures in Example 14 was cut out to a piece 8 mm in diameter, which was placed on the medium with the organism applied and incubated at 37°C for 18 hours. The results are shown in Table 2.

**Table 2**

| Antimicrobial activity of the antibially active wound-covering materials | | | | |
|---|---|---|---|---|
| Microorganism | Test material | | | |
| | PP-Ag(200W*) -PMEA | PP-Ag(400W*) -PMEA | PP-Ag(800W*) | PP-PMEA |
| Psudomonas aeruginosa | (+) 0.5 mm | (+) 1 mm | (+) 2 mm | (-) |
| Staphylococcus | (+) 0.5 mm | (+) 1 mm | (+) 0.5 mm | (-) |

| | | | | |
|---|---|---|---|---|
| * Power output of the electric source in Ag sputtering. Added amount of Ag increases in proportion to the power output. | | | | |
| (+) Antimicrobially active (-) Antimicrobially inactive | | | | |

The figure indicates diameter of the inhibition zone.

### Test Example 2

Antimicrobial activity was assessed in the same way as in Test Example 1 for the wound-covering materials prepared in Example 11 and the wound-covering materials of porous polypropylene membrane with silver vapor deposited but without hydrophilic polymer. The results are shown in Table 3.

**Table 3**

| Microorganism | Example | | Comparative Example | |
|---|---|---|---|---|
| | PP-Ag(200W) -PMEA | PP-Ag(400W) -PMEA | PP-Ag(200W) | PP-Ag(400W) |
| Psudomonas aeruginosa | (+) 0.5 mm | (+) 1 mm | (+) 0.5 mm | (+) 0.5 mm |
| Staphylococcus | (+) 0.5 mm | (+) 0.5 mm | (-) | (-) |

Thus the wound-covering materials of the invention, when applied to the affected part caused by disease or wound such as burn, wound caused by removal of skin for dermatoplasty, avulsed wound, excoriation and traumatic dermal defective wound, can be in close contact with the wound surface and facilitate regeneration of the epidermis due to adequate moisture permeability and wetting of the contacted surface. Particularly, infection of the wound can be prevented by carrying a metal provided with antimicrobial activities.

Bactericidal performances are improved by having a hydrophilic polymer and an antibially active metal simultaneously present on the surface of the wound-covering material as compared to the absence of the hydrophilic polymer. This improvement in antimicrobial activity may presumably be due to loosed structure by swelling with water of the hydrophilic polymer introduced upon the surface of the covering material which facilitates release and diffusion of metal ions.

## Claims

1. A wound-covering material comprising a hydrophobic porous membrane, a surface and inner surfaces of the pores of the hydrophobic membrane being coated with a nonionic surface active agent or an amino-acid surface-active agent, the hydrophobic membrane being composed of a polyolefin or a halogenated polyolefin, the nonionic surface active agent being a propylene oxid/ethylene oxide block copolymer in which 60-90% by weight of the whole molecule is suitably occupied by an ethylene oxide chain, the amino-acid surface active agent being anionic and containing as a hydrophilic group an amino acid and as a hydrophobic group a fatty acid.

2. The wound-covering material according to claim 1 wherein a pore diameter of the hydrophobic porous membrane is 0.01-1.0 »m on average.

3. A wound-covering material comprising a hydrophobic membrane composed of a porous polyolefin or halogenated polyolefin and a surface of the membrane being chemically bound with a methoxyethyl acrylate or N-dimethylacrylamide.

4. The wound-covering material according to claim 3 wherein a pore diameter of the hydrophobic porous membrane is 0.01-1.0 »m on average.

5. The wound-covering material according to claim 3 wherein steam permeability coefficient of the membrane is 200-5000 mg/m²/24 hrs.

6. The wound-covering material according to claims 3 - 5 additionally comprising an antimicrobially active metal vapor-deposited on the surface of the membrane.

## Patentansprüche

1. Wundabdeckmaterial mit einer hydrophoben porösen Membran, wobei die Oberfläche und die Poreninnenflächen der hydrophoben Membran mit einem nichtionischen grenzflächenaktiven Mittel oder einem eine Aminosäure umfassenden grenzflächenaktiven Mittel beschichtet sind, die hydrophobe Membran aus einem Polyolefin oder einem halogenierten Polyolefin besteht, das nichtionische grenzflächenaktive Mittel ein Propylenoxid/Ethylenoxid-Blockcopolymer ist, in dem in geeigneter Weise eine Ethylenoxidkette 60 - 90 Gew.-% des Gesamtmoleküls ausmacht, und das eine Aminosäure umfassende grenzflächenaktive Mittel anionisch ist und als eine hydrophile Gruppe eine Aminosäure und als eine hydrophobe Gruppe eine Fettsäure enthält.

2. Wundabdeckmaterial nach Anspruch 1, wobei der Porendurchmesser der hydrophoben porösen Membran im Mittel 0,01 bis 1,0 »m beträgt.

3. Wundabdeckmaterial mit einer hydrophoben Membran aus einem porösen Polyolefin oder halogenierten Polyolefin, wobei eine Oberfläche der Membran chemisch an ein Methoxyethylacrylat oder N-Dimethylacrylamid gebunden ist.

4. Wundabdeckmaterial nach Anspruch 3, wobei der Porendurchmesser der hydrophoben porösen Membran im Mittel 0,01 bis 1,0 »m beträgt.

5. Wundabdeckmaterial nach Anspruch 3, wobei der Dampfpermeabilitätskoeffizient der Membran 200 bis 5.000 mg/m²/24 h beträgt.

6. Wundabdeckmaterial nach Ansprüchen 3 bis 5, das zusätzlich ein auf der Oberfläche der Membran durch Dampfabscheidung aufgebrachtes anti-mikrobiell wirksames Metall aufweist.

## Revendications

1. Matériau de recouvrement de plaies comprenant une membre poreuse hydrophobe, une surface et les surfaces intérieures des pores de la membrane hydrophobe étant revêtues d'un agent tensioactif non ionique ou d'un agent tensio-actif de type acide aminé, la membrane hydrophobe étant constituée d'une polyoléfine ou d'une polyoléfine halogénée, l'agent tensioactif non ionique étant un copolymère séquencé d'oxyde de propylène/oxyde d'éthylène dans lequel 60 à 90% en masse de toute la molécule sont occupés de manière adaptée par une chaîne d'oxyde d'éthylène, l'agent tensioactif de type acide aminé étant anionique et contenant, en tant que groupe hydrophile, un acide aminé et, en tant que groupe hydrophobe, un acide gras.

2. Matériau de recouvrement de plaies selon la revendication 1, dans lequel le diamètre de pore de la membrane poreuse hydrophobe est compris en moyenne entre 0,01 et 1,0 »m.

3. Matériau de recouvrement de plaies comprenant une membrane hydrophobe constituée d'une polyoléfine poreuse ou d'une polyoléfine halogénée poreuse, une surface de la membrane étant chimiquement liée à de l'acrylate de méthoxyéthyle ou à du N-diméthylacrylamide.

4. Matériau de recouvrement de plaies selon la revendication 3, dans lequel le diamètre de pore de la membrane poreuse hydrophobe est compris en moyenne entre 0,01 et 1,0 »m.

5. Matériau de recouvrement de plaies selon la revendication 3, dans lequel le coefficient de perméabilité à la vapeur de la membrane est compris entre 200 et 5 000 mg/m²/24h.

6. Matériau de recouvrement de plaies selon les revendications 3 à 5, comprenant de plus un métal à activité anti-microbienne déposé en phase vapeur sur la surface de la membrane.
